# EUROPEAN PATENT APPLICATION

(11) **EP 2 308 484 A1**
(43) Date of publication of application: **13.04.2011**
(21) Application number: 09172364.3
(22) Date of filing: 06.10.2009
(51) Int. Cl.: A61K 31/18, A61P 37/06

(54) **Inhibitors of cxcr1/2 as adjuvants in the transplant of pancreatic islets**

(71) Applicant: Dompé S.p.a., 67100 L'aquila (IT)
(72) Inventor: Piemonti, Lorenzo, 67100, L`Aquila AQ (IT); Daffonchio, Luisa, 67100, L`Aquila AQ (IT); Allegretti, Marcello, 67100, L`Aquila AQ (IT)
(74) Representative: Perani, Aurelio

(57) **Abstract**

The invention relates to CXCR1 and CXCR2 inhibitors for the preparation of a medicament for use as an adjuvant in the transplant of pancreatic islets in Type 1 diabetes patients. In particular, the compounds thagt can be used according to the invention have the following formula (I) 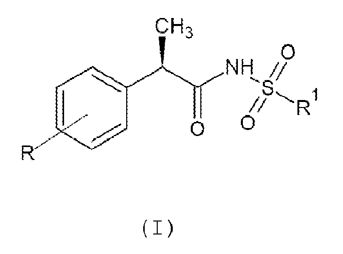 in which R and R' are as defined in the description.

## Description

### Field of the invention

The present invention relates to compounds useful as adjuvants in the transplant of pancreatic islets in Type 1 diabetes patients.

### Background of the invention

Transplantation of pancreatic tissue, in form of the whole pancreas or of isolated pancreatic islets, has become a clinical option in the treatment of Type 1 insulin-dependent diabetes mellitus.

Pancreatic islet transplantation is particularly attractive since it is a less invasive alternative compared to whole pancreas transplantation and it is associated with a much lower risk of serious complications.

However, such a procedure is still limited by the poor efficiency.

The early strategies of islet transplantation were based on protocols that had proven successful in solid organ transplantation and comprised the administration of immunosuppressive agents such as azathioprine, cyclosporine and corticosteroids. Such strategies demonstrated not to be effective in the specific case of pancreatic islet transplantation and were associated to very poor results, with most of the grafts failing within one year from transplant (Sulaiman and Shapiro, Diabetes, Obesity and Metabolism, 8, 2006, 15-25).

In recent years, the development of the Edmonton protocol, which has introduced new specific immunosuppressive regimes and islet preparation techniques, has dramatically improved the clinical outcome of islet transplantation.

According to the Edmonton protocol, pancreatic islets are isolated from the pancreas of a deceased donor, purified and then transplanted in a recipient by means of a catheter placed through the upper abdomen and into the portal vein of the liver; soon after their infusion into the liver, the cells begin to release insulin. In order to prevent rejection, a new immunosuppressive regime is used that requires the use of a combination of immunosuppressive drugs, namely Sirolimus and Tacrolimus and of a CD25 monoclonal antibody, Daclizumab (Saphiro et al. N Engl. J Med, 2000, 343(4):230-238).

Unfortunately, there are still some flaws of islet trasplantation that have not been solved and that prevent this procedure to become the standard treatment for patients with Type 1 diabetes.

A first drawback associated to pancreatic islet transplantation is that, even if the Edmonton protocol has significantly increased the rate of success, there is still a high percentage of early graft failure due to a series of complex phenomena such as IBMIR, recruitment of inflammatory cells and aspecific immunity. In fact, intrahepatic islet infusion in men is associated with an immediate blood-mediated inflammatory reaction, thrombosis and hepatic tissue ischemia with elevated blood liver enzymes (Barshes NR at al., J Am Coll Surg, 2005, 200(3): 353-361; Barshes NR et al, J Leukoc Biol, 2005, 77(5):587-97; Bertuzzi et al, J Clin Endocrinol Metab, 2004, 89(11): 5724-8; Bhargava R et al Diabetes, 2004, 53(5),: 1311-7; Contreras et al, 2004, 53(11):2894-14; Johansson et al, Diabetes, 2005, 54(6):1755-62). Loss of as many as 50-75% of islets during engraftment in the liver (Contreras et al, see above) has been suggested to be the main factor responsible for the huge number of islets needed to achieve normoglycemia (Barshes et al, see above).

Furthermore, even when the transplantation is initially successful and leads to insulin independence of the recipient, the transplanted islets seem to loose their ability to function over time. This event limits the possibility to achieve long-lasting insulin independence in the transplanted patients, with only 14% of the patients showing insulin independence after 2 years from the transplant [Meloche RM World J Gastroenterol 2007; 13 (47) : 6347-6355].

A further drawback is that the Edmonton protocol requires the use of a combination of immunosuppressive drugs. Sirolimus and Tacrolimus must be taken for life or for as long as the transplanted islets continue to function. However, these drugs have significant side-effects, which would be desirable to reduce. Complications deriving from the immunosuppression therapy are the second most common severe event reported in this type of transplant.

Thus, further developments are still necessary to improve the long-term viability and function of the graft to maintain glucose control over time and to reduce immunosuppressive therapy.

CXCL8 is a chemokine inducible by inflammatory mediators that is been implicated in early phases of tissue repair and that has been demonstrated to promote angiogenesis (Li et al, J Immunol, 2003, 170: 3369-3376) through induction of chemotaxis, survival and proliferation of endothelial cells, and to act as a neutrophil attractant. It exerts its action by binding to its cognate G-protein coupled receptors CXCR1 and CXCR2.

Recent literature has hypothesized that CXCL8 may promote engraftment through the induction of revascularization of the grafted tissue (Movahedi et al, Diabetes, 2008, 57: 2128-36).

EP 1 123 276 discloses N-(2-aryl-propionyl)-sulfonamides, among them R(-)-2-[(4-isobutylphenyl)propionyl]-methanesulfonamide (I) and their pharmaceutically acceptable salts, for use as inhibitors of neutrophil chemotaxis and degranulation induced by CXCL-8, in particular for use in the treatment of pathologies like psoriasis, rheumatoid arthritis, ulcerative colitis, acute respiratory insufficiency (ARDS), idiopathic fibrosis and glomerulonephritis.

EP 1 355 641 discloses the use of R(-)-2-[(4-isobutylphenyl)propionyl]-methanesulfonamide and pharmaceutically acceptable salts thereof, in particular its lysine salts, in the prevention and treatment of ischemia/reperfusion injury of transplanted organs and of functional injury resulting from rejection reactions after solid organ transplantation, in particular kidneys, which need to be retrieved from a donor and stored before transplantation. Such injuries are deemed to be responsible for delayed graft function, which makes dialysis necessary in case of renal transplantation.

EP 1 579 859 discloses the use of N-(2-aryl-propionyl)-sulfonamides, among them R(-)-2-[(4-isobutylphenyl)propionyl]-methanesulfonamide and its lysine salt, for the preparation of medicaments for the treatment of spinal cord injury.

### Description of the Figures

Figure 1: Panel A represents non fasting glycemia (in mg/dl) measured from day-1 to day +7 after isotransplantation of 400 pancreatic islets in knock out (faded line) and wild type (black line) mice. Panel B represents the results of Oral Glucose Tolerance Test (OGTT). Glycemia (in mg/dl) was measured immediately before administration of glucose and after 10, 20, 30, 60 and 90 minutes after administration of oral glucose. The curve of blood glucose is shown per each animal.
Figure 2: Panel A represents glycemia at different time courses after transplant in Reparixin treated (solid line) or control (dotted line) mice. Panel B represents Cox regression multivariate analysis.

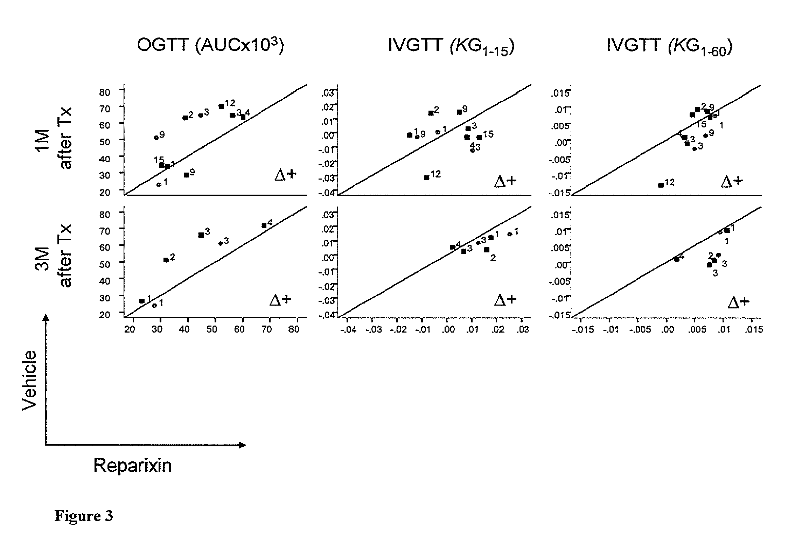

Figure 3 reports in scatter plots, each representing the mean value obtained in the Oral Glucose Tolerance Test (OGTT) and in the Intravenous Glucose Tolerance Test (IVGTT) in mice transplanted in the presence or in the absence of Reparixin with islets from the same isolation. Labels identify the number of the isolation. Squares and circles represent respectively mice transplanted with 250 or 150 IE. Upper and lower panels report the data respectively 1 and 3 months after transplantation. The solid line is the identity line: circles above the identity line represent observations with higher values in the Reparixin group than in the vehicle-treated group (Δ+).
Figure 4 represents the circulating levels of alanine aminotransferase (ALT) 24h and 48h after Tx in Reparixin and vehicle treated animals transplanted with 150 (Panel A) or 250 IE (Panel B).
Figure 5: Panel A represents glycemia at different times after transplant in mice treated with Reparixin, Rapamycin, Reparixin + Rapamycin or Vehicle. Panel B represents Cox regression multivariate analysis.
Figure 6: circulating levels of alanine aminotransferase (ALT) 24h and 48h after Tx in mice treated with vehicle (A), Reparixin (B), Rapamycin (C) or Reparixin + Rapamycin (D).
Figure 7: Panel A represents the percentage of transplant survival over time after transplant in mice treated with Reparixin, Rapamycin, Reparixin + Rapamicyn or Vehicle. Panel B represents Cox regression multivariate analysis.

### Detailed description of the invention

The present inventors have now surprisingly found that, contrary to what expected from the prior art teachings, agonists of CXCR1 and CXCR2 are detrimental for islet survival following pancreatic islet transplantation. As it will be described in the following Examples, pancreatic islets show an enhanced function and survival when they are transplanted in CXCR2 knock out BALB/C mice compared to the wild type mice, with a consistent better glucose tolerance and lower glucose concentration than control mice.

Furthermore, experiments carried out by the present inventors clearly demonstrate that compounds that inhibit CXCR1 and CXCR2 signalling are able to effectively improve graft survival and function following pancreatic islet transplantation.

Accordingly, a first object of the present application is the use of inhibitors of CXCR1 and CXCR2 as adjuvants in the transplant of pancreatic islets in Type 1 diabetes patients.

For "inhibitors of CXCR1 and CXCR2" according to the present invention it is meant compounds that are able to prevent CXCL8-biological activity derived from CXCR1 and CXCR2 activation. These compounds may be competitive antagonists or allosteric inhibitors of the receptors.

Preferred compounds of the invention are compounds of formula I, or pharmaceutically acceptable salts thereof: 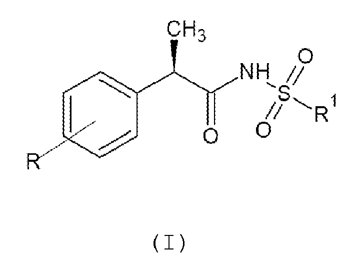 wherein R is selected from linear or branched 4-(C₁-C₆) alkyl, 4-trifluoromethanesulfonyloxy or 3-benzoyl and R¹ is linear or branched (C₁-C₆)alkyl, and, in particular, R(-)-2-[(4-isobutylphenyl)propionyl]-methanesulfonamide.

A particularly preferred salt of the compounds of the invention is the lysine salt.

As will be described hereinbelow, in animal models the compounds of formula I are able to effectively improve graft survival and function following pancreatic islet transplantation.

In detail, data obtained in experimental models of islet transplantation demonstrate a clear effect of the above compounds, represented by R(-)-2-[(4-isobutylphenyl)propionyl]-methanesulfonamide (commonly known as Repertaxin or Reparixin), in the protection from the loss of activity and/or deterioration of the transplanted β-cells.

According to a preferred embodiment of the invention, said compound of formula I is Reparixin.

The compounds of the invention are effective in supporting the engraftment of transplanted pancreatic islet cells in Type 1 diabetes.

Indeed, as it will be clearer from the following experimental section, intravenous administration of Reparixin in animal models from day -1 to day +6 after syngenic or allogenic pancreatic islets transplant resulted in a higher probability and in a reduced median time to reach normal levels of glycemia (a non-fasting blood glucose level lower than 250 mg/ml) compared to the controls.

Thus, a further object of the present invention is the use of the compounds of the invention, preferably of Reparixin, for improving engraftment and early graft function and for reducing the occurrence of early graft failure following transplant of pancreatic islets in Type 1 diabetes patients.

Experiments of allogenic islets transplantation demonstrated that the administration of Reparixin significantly reduced the occurrence of rejection reactions in those mice achieving primary function post transplant.

Furthermore, the results demonstrate that graft function is maintained for a longer time compared to the controls, with an increase of the median survival time.

Thus, a further object of the present invention is the use of the compounds of formula (I), preferably of Reparixin, for reducing graft rejection reactions and for improving long term graft survival.

The compounds of the invention can be used to this aim alone or in a combination therapy with one or more immunosuppressants, preferably selected from Sirolimus (also known as Rapamycin) and Tacrolimus.

However, the data obtained also suggest that the use of a compound of the invention alone may be sufficient to inhibit graft rejection. Indeed, as shown in the experimental section, Reparixin significantly increased the time of rejection in mice that achieved primary function post-transplant while this was not significantly varied by Rapamycin. Furthermore, administration of Reparixin alone provided results comparable to those obtained by administration of a combination of Reparixin with Rapamycin. Therefore, these data strongly suggest that administration of Reparixin alone may be sufficient in order to reduce the graft rejection reaction, without the need or with a reduced need of an immunosuppressive therapy, which is a remarkable advantage in terms of toxicity.

The synthesis of the compounds of the invention may be carried out according to procedures well known in the art. For example, Reparixin can be prepared as disclosed in Example 1 of EP 1 123 276 and in example 1 of EP 1 355 641, while the lysine salt can be prepared as disclosed in example 7 and example 2 respectively of the aforementioned patents.

For the purposes of the present invention the compounds of formula I or their pharmaceutically acceptable salts are formulated in pharmaceutical compositions suitable for use by oral administration, such as tablets capsules, syrups, preferably in the form of controlled release formulations, or by parenteral administration, preferably in the form of sterile solutions suitable for intravenous or intramuscular administration. The pharmaceutical forms can be prepared according to conventional methods, for example as disclosed in Remington, "The Science and Practice of Pharmacy", 21st ed. (Lippincott Williams and Wilkins). Preferably, the amount of R (-) -2- [ (4-isobutylphenyl) propionyl] - methanesulfonamide or its pharmaceutically acceptable salt in each of the above-mentioned administration forms will be such as to provide between 2 and 15 mg compound or salt/kg body weight. In any case, the regimen and amount of medicament to be administered will be determined by the physician according to the patient's need.

The invention will be now further illustrated in greater details in the following experimental section.

### EXPERIMENTAL SECTION

### 1. Syngeneic islet transplantation in knock out mice

In order to test the role of activation of CXCL8 signalling pathway through CXCR1 and CXCR2 on islet survival, islet function after syngeneic islet intrahepatic transplant was evaluated in Balb/c CXCR2-/- mice and CXCR2+/+ mice. CXCR1 is not expressed in mice and thus knock out of CXCR2 totally abolishes the signalling induced by CXCL8.

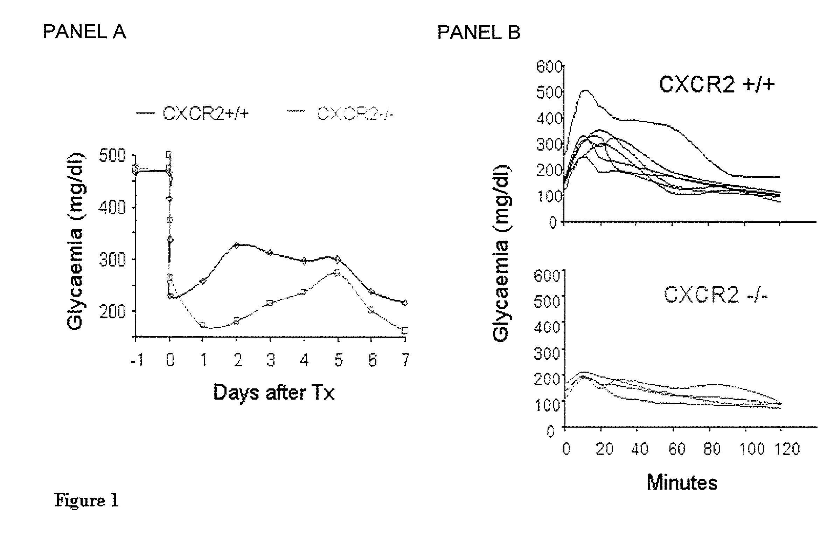

Non fasting glycemia during the first week after transplant and oral glucose tolerance 4 weeks after transplant were taken as indicators of functionality. As reported in Figure 1, transplanted CXCR2 knock out mice clearly showed a consistently better glucose tolerance than control wild type mice, as demonstrated by the significant reduction of circulating glucose concentration during the whole period of evaluation.

Oral glucose tolerance test was conducted as described below, in section 2.

### 2. Syngeneic islet transplantation in mice

Islets from 12 week old C57 mice were transplanted in the liver of diabetic C57 mice (alloxan induced diabetes, glycaemia >450 mg/dl). Two different marginal islet mass models, 150 IE (Islet Equivalents) and 250 IE, were used. Reparixin was administered by s.c. continuous infusion starting from day -1 up to day 6 or 13 after islet transplantation at a dose of 8 mg/kg/h. Control animals received continuous s.c. vehicle.

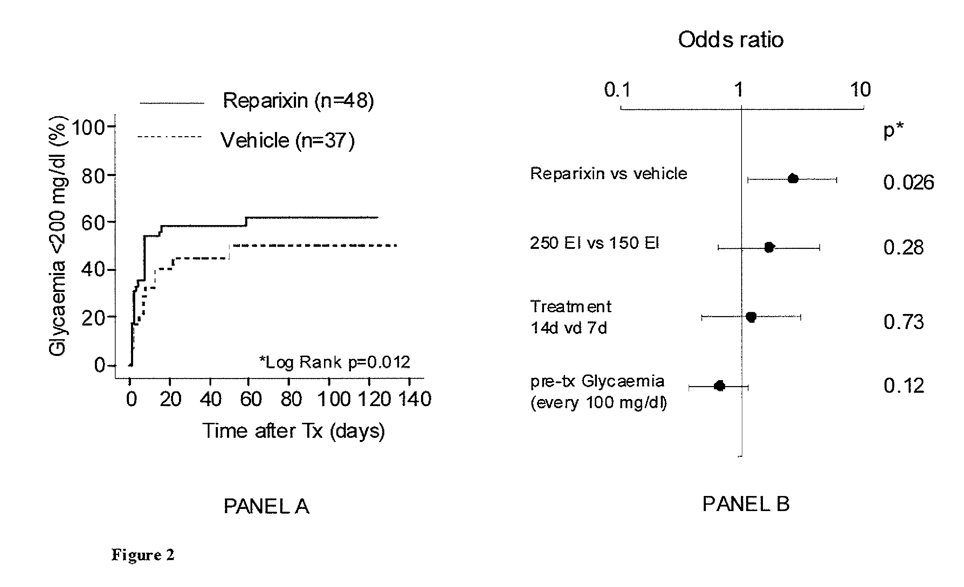

The ability to reach a non-fasting blood glucose level less than 200 mg/dl for two consecutive measurements after islet transplantation was first evaluated. As shown in Figure 2 the probability and median time to reach euglycaemia (<200 mg/dl) were: 50% and 7 days for mice treated with Reparixin as compared to 35.1% and 50 days for mice treated with vehicle (Log Rank p<0.012).

A Multivariate Cox Regression Analysis, in which Reparixin treatment, duration of the treatment, number of transplanted islet and recipient pre-transplant glycaemia were included as covariates, confirmed that the outcome was significantly improved by Reparixin (Odds ratio: 2.6; 95% CI: 1.1-6.1; p<0.021). As expected, transplantation of 250 IE resulted in an improved grafting (Odds ratio: 1.6; 95% CI: 0.6-4.3; p<0.28), while pre-transplant glycemia negatively affected the outcome (Odds ratio:0.6, 95%CI: 0.3-1.1; p=0.12).

An intravenous glucose tolerance test (IVGTT) and an oral glucose tolerance test (OGTT) were performed 1 and 3 months after transplantation to evaluate the functionality of the grafted islets. IVGTT was initiated after a 16-hour fast; mice were given glucose (0.5 g/kg) by tail vein injection. Blood samples were obtained 0, 1, 5, 15, 20, 30 and 60 minutes after injection and were used to determine glucose concentrations. From the IVGTT, glucose tolerance was quantified from the glucose elimination constant (KG; expressed as percent elimination of glucose per minute) as the reduction in circulating glucose between 1 and 15 min (KG₁₋₁₅) after intravenous administration, following the logarithmic transformation of the individual plasma glucose values. A similar estimation was performed for the total 1-to 60-min glucose disappearance rate (KG ₁₋₆₀). This parameter indicates the rate of glucose disappearance during the whole test. OGTT was initiated after a 4-hour fast; mice were given glucose (1 g/kg) by oral gavage. Blood samples were obtained 0, 10, 20, 30, 60, 90 and 120 minutes after glucose administration and used to determine glucose concentrations. The area under the curve (AUC) for glucose during OGTT was calculated using the trapezoidal method (baseline = 0 min). The effects of islet transplantation on glucose tolerance after IVGTT and OGTT are illustrated in Figure 3. The data are reported as scatter plots. Each point represents the mean value of the measured parameter in mice transplanted in the presence ○ in the absence of Reparixin with islets from the same isolation. Labels identify the isolation number. Squares and circles represent respectively mice transplanted with 250 or 150 IE. Upper and lower panels respectively report the data 1 and 3 months after transplantation. The solid line is the identity line: circles above the identity line represent observation with higher values in the Reparixin-treated group than in the vehicle-treated group (Δ+). OGTT 1 and 3 months post transplant showed that in mice treated with Reparixin the AUC for glucose remains lower than that of the control mice. In keeping with these data, the glucose elimination constants between 1 and 15 min (*KG*₁₋₁₅) and 1 and 60 min (*KG*₁₋₆₀) were significantly increased in Reparixin-treated mice as compared to control mice.

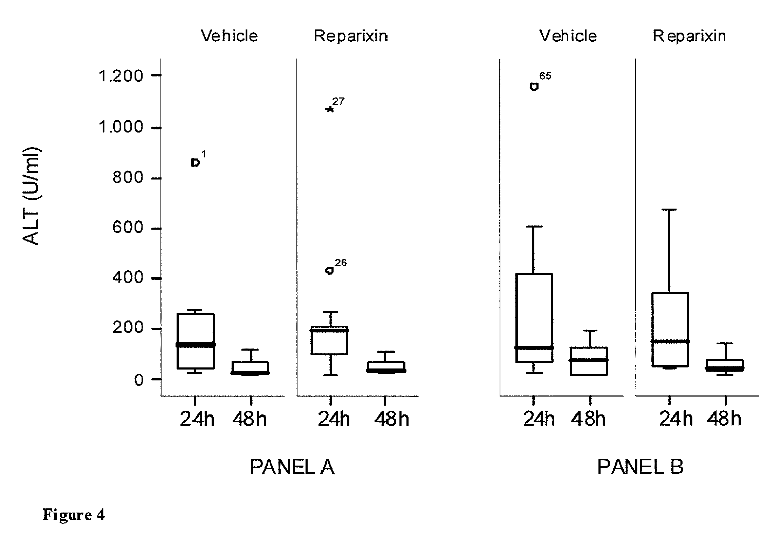

Acute liver damage was quantified by circulating levels of alanine aminotransferase (ALT) 24h and 48h after Transplantation (Tx). ALT levels were not affected by Reparixin treatment both in mice transplanted with 150 or 250 IE (Figure 4). Similarly, no difference was evident in circulating levels of white blood cells, red blood cells and platelets (data not shown).

### 3. Allogeneic islet transplantation in mice

Islets from 12 week old Balb/c mice were transplanted in the liver of diabetic C57 mice (alloxan, glycaemia >450 mg/dl). In some experiments, islets from 12-week-old C57BL/6(B6) mice were transplanted in the liver of diabetic female NOD/LtJ (NOD) mice in order to evaluate the presence of any autoimmune reaction. NOD mice were used as recipients of islet transplant after at least three non-fasting blood glucose readings higher than 350 mg/dL. In both cases 400 IE were transplanted. Animals were treated with Reparixin alone (5.28 mg/kg/h continuous s.c. infusion starting from day -1 up to day 7 after transplant), Rapamycin alone (daily i.p. injections starting with an induction dose of 0.3 mg/kg on day 0 followed by a maintenance dose of 0.15 mg/kg until day 14), Reparixin + Rapamycin or vehicle.

The ability to reach primary function, defined as non-fasting blood glucose levels less than 250 mg/dl for 2 consecutive measurements after islet transplantation and the time to rejection defined as 2 consecutive non-fasting blood glucose readings greater than 300 mg/dL were first evaluated.

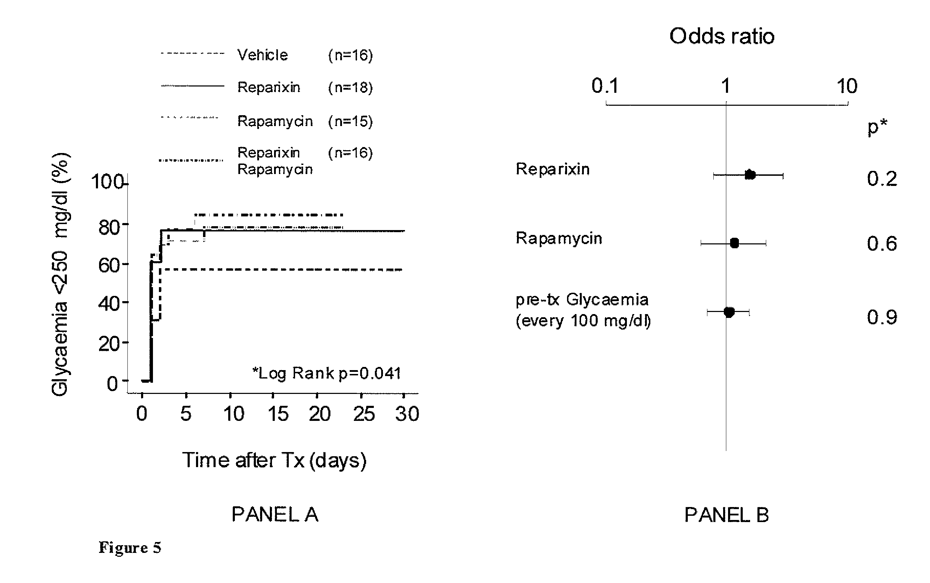

Considering all the mice transplanted in the alloimmune setting, the probability and the median time to reach the primary function (glycaemia <250 mg/dl) were: 72% and 1 day for mice treated with Reparixin alone, 73% and 1 day for mice treated with Rapamycin alone, 69% and 1 day for mice treated with Reparixin + Rapamycin, 44% and 2 days for mice treated with vehicle, (Figure 5), Log Rank p<0.041). A Multivariate Cox Regression Analysis in which Reparixin treatment, Rapamycin treatment and recipient pre-transplant glycaemia were included as covariates, confirmed the outcome improvement by Reparixin treatment(Odds ratio: 2.5; 95% CI: 0.79-2.99; p<0.202). Treatment with Rapamycin (Odds ratio: 1.1; 95% CI: 0.62-2.15; p<0.62) and pre-transplant glycaemia (Odds ratio: 1.03; 95% CI: 0.68-1.59; p<0.88) were less relevant.

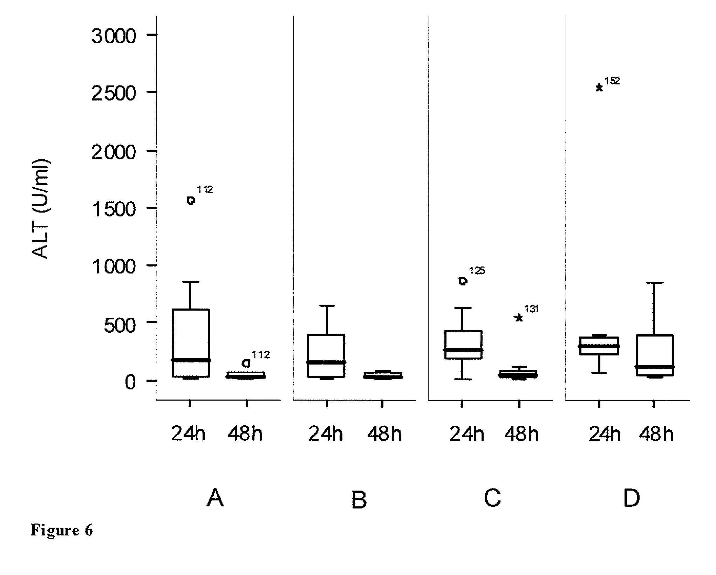

Circulating levels of ALT measured 24h and 48h after transplant were not affected by Reparixin both in the presence and in the absence of Rapamycin (Figure 6).

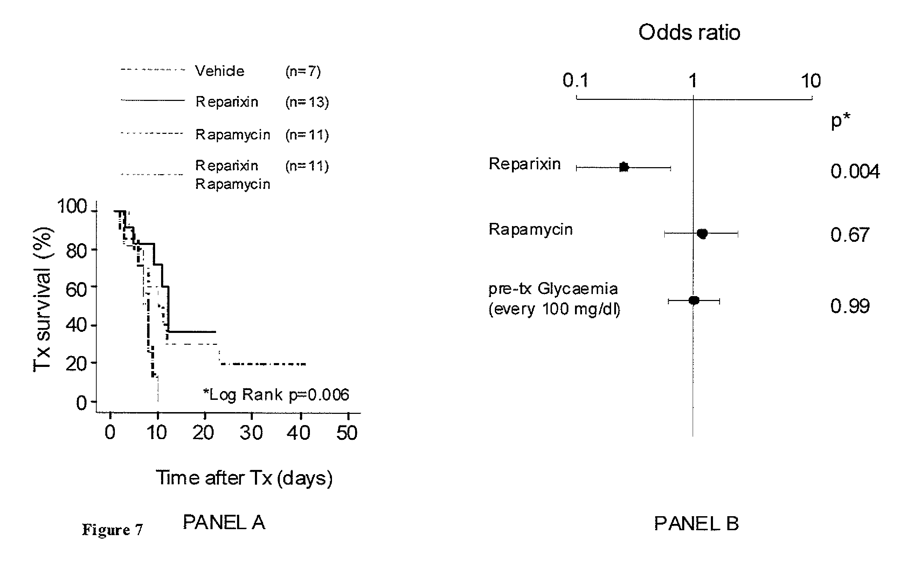

Besides, treatment with Reparixin significantly increased the time to rejection in mice that achieved primary function post-transplant (Figure 7). The median survival time was 12±0.6 days (n=13) and 8±0.5 days (n=7) respectively for Reparixin and vehicle treated mice in the absence of Rapamycin. In the presence of Rapamycin the median survival time was 12±2 days (n=11) and 8±0.6 days (n=11) respectively for Reparixin and control mice. Data were confirmed by a Multivariate Cox Regression Analysis, including Reparixin treatment, Rapamycin treatment and recipient pre-transplant glycaemia as covariates.

Treatment with Reparixin was confirmed as a significant independent protection factor for the loss of graft survival (Odds ratio: 0.252; 95% interval of confidence: 0.099-0.64; p=0.004) while Rapamycin treatment (Odds ratio: 1.173; 95% interval of confidence: 0.562-2.45; p=0.67) and the pre-transplant glycaemia (Odds ratio: 1.002; 95% interval of confidence: 0.604-1.661; p=0.99) were not significant.

## Claims

1. Use of an inhibitor of CXCR1 and CXCR2 for the preparation of an adjuvant medicament for the transplant of pancreatic islets in Type 1 diabetes patients.

2. The use as claimed in claim 1 wherein said inhibitor is a compound of formula I, or a pharmaceutically acceptable salt thereof: 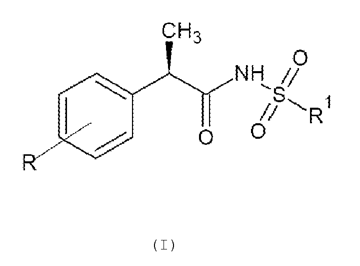 wherein R is selected from linear or branched 4-(C₁-C₆) alkyl, 4-trifluoromethanesulfonyloxy and 3-benzoyl and R' is linear or branched (C₁-C₆) alkyl.

3. The use according to claim 2 in which the pharmaceutically acceptable salt is the lysine salt.

4. The use according to any one of claims 1 to 3, wherein said medicament improves pancreatic islet cells engraftment and early graft function.

5. The use according to any one claims 1 to 4, wherein said medicament reduces the occurrence of failure of transplanted pancreatic islets.

6. The use according to claim 5 wherein said medicament reduces the time and occurrence of pancreatic islet cells graft rejection.

7. The use according to claim 6 wherein said medicament improves long term graft survival.

8. The use according to any one of claims 1 to 7 wherein said compound of formula I is R(-)-N-2-[(4-isobutylphenyl)propionyl]-methanesulfonamide.
